# EUROPEAN PATENT SPECIFICATION

(11) **EP 4 138 828 B1**
(45) Date of publication and mention of the grant of the patent: **01.10.2025**
(21) Application number: 21725987.8
(22) Date of filing: 20.04.2021
(51) Int. Cl.: A61K 31/4415, A61K 31/7076, A61K 33/08, A61K 35/745, A61K 35/747, A61K 35/741, A61K 9/00, A61K 9/16, A61K 9/20, A61P 1/06, A61P 25/22, A61P 25/24

(54) **ORAL COMPOSITION CONTAINING S-ADENOSYLMETHIONINE (SAMe) AND A MIXTURE OF PROBIOTICS AND USE THEREOF**
ORALE ZUSAMMENSETZUNG ENTHALTEND S-ADENOSYLMETHIONIN (SAMe) UND EINE MISCHUNG VON PROBIOTIKA, UND IHRE VERWENDUNG
COMPOSITION ORALE CONTENANT DE LA S-ADÉNOSYLMÉTHIONINE (SAMe) ET UN MÉLANGE DE PROBIOTIQUES ET SON UTILISATION

(30) Priority: 21.04.2020 IT 202000008482
(43) Date of publication of application: 01.03.2023
(73) Proprietor: MONTEFARMACO OTC S.p.A., 20021 Bollate (MI) (IT); Graal S.r.l., 20900 Monza (MB) (IT)
(72) Inventor: COLOMBO, Stefano, 20021 Bollate (MI) (IT); SENECI, Antonio Enrico, 20122 Milano (MI) (IT)
(74) Representative: Bird & Bird Società tra Avvocati S.r.l.
(86) International application number: PCT/IB2021/053258
(87) International publication number: WO 2021/214661

(56) References cited:
- WO-A1-2010/089674
- WO-A1-2016/065419
- WO-A1-2018/002238
- CN-A- 105 213 433
- US-B1- 6 368 617
- ULLAH HAMMAD ET AL: "The Efficacy of S-Adenosyl Methionine and Probiotic Supplementation on Depression: A Synergistic Approach", NUTRIENTS, vol. 14, no. 13, 1 July 2022 (2022-07-01), CH, pages 2751, XP093116429, ISSN: 2072-6643, Retrieved from the Internet <URL:https://pubmed.ncbi.nlm.nih.gov/35807931/> DOI: 10.3390/nu14132751
- BUONOCORE DANIELA ET AL: "Assorbimento di S-Adenosil-L-Metionina (SAM-e) formulata in un innovativo granulato orosolubile", L'INTEGRATORE NUTRIZIONALE, 25 October 2022 (2022-10-25), pages 18 - 23, XP093116432, Retrieved from the Internet <URL:https://www.ceceditore.com/lintegratore-nutrizionale-5-2022/> [retrieved on 20240107]
- ULLAH HAMMAD ET AL: "Efficacy of a food supplement based on S-adenosyl methionine and probiotic strains in subjects with subthreshold depression and mild-to-moderate depression: A monocentric, randomized, cross-over, double-blind, placebo-controlled clinical trial", BIOMEDICINE & PHARMACOTHERAPY, vol. 156, 1 December 2022 (2022-12-01), FR, pages 113930, XP093116433, ISSN: 0753-3322, Retrieved from the Internet <URL:https://pubmed.ncbi.nlm.nih.gov/36411659/> DOI: 10.1016/j.biopha.2022.113930
- ANONYMOUS: "EVALUATION OF EFFICACY OF SAMEUP IN SUBJECTS WITH DEPRESSION SYMPTOMS: A RANDOMIZED STUDY (SAMEUP)", CLINICALTRIALS.GOV, 2 May 2019 (2019-05-02), pages 1 - 8, XP055756279, Retrieved from the Internet <URL:https://www.clinicaltrials.gov/ct2/show/NCT03932474?term=nct03932474&draw=2&rank=1> [retrieved on 20201203]
- MICHAËL MESSAOUDI ET AL: "Assessment of psychotropic-like properties of a probiotic formulation ( Lactobacillus helveticus R0052 and Bifidobacterium longum R0175) in rats and human subjects", BRITISH JOURNAL OF NUTRITION, vol. 105, no. 05, 26 October 2010 (2010-10-26), pages 755 - 764, XP055192798, ISSN: 0007-1145, DOI: 10.1017/S0007114510004319
- ARSLAN-TONTUL SULTAN ET AL: "Single and double layered microencapsulation of probiotics by spray drying and spray chilling", LWT- FOOD SCIENCE AND TECHNOLOGY, vol. 81, 2017, pages 160 - 169, XP029991590, ISSN: 0023-6438, DOI: 10.1016/J.LWT.2017.03.060
- SACCARELLO ALBERTO ET AL: "Oral Administration of S-Adenosylmethionine (SAMe) and Lactobacillus Plantarum HEAL9 Improves the Mild-To-Moderate Symptoms of Depression : A Randomized, Double-Blind, Placebo-Controlled Study", THE PRIMARY CARE COMPANION TO THE JOURNAL OF CLINICAL PSYCHIATRY : THE OFFICIAL JOURNAL OF THE ASSOCIATION OF MEDICINE AND PSYCHIATRY, vol. 22, no. 4, 25 June 2020 (2020-06-25), USA, XP055821458, ISSN: 1523-5998, Retrieved from the Internet <URL:http://dx.doi.org/10.4088/PCC.19m02578> DOI: 10.4088/PCC.19m02578

## Description

The present invention refers to an orosoluble solid oral composition comprising the combination of S-adenosyl methionine with a mixture of probiotics consisting of Lactobacillus helveticus Rosell^{®}-52 and Bifidobacterium longum Rosell^{®}-175, in which said probiotics are present in the combination in a microencapsulated form, at least one physiologically acceptable excipient and an additional agent selected from a mixture of magnesium oxide and vitamin B6.

In particular, the invention refers to said orosoluble oral composition for use in the treatment and/or prevention of mood disorders, preferably for the treatment of anxiety, depression, psychological stress, mild postpartum syndromes and/or stress related intestinal disorders.

Preferably said depression is a minor subthreshold depression (*mild*-*depression*).In a preferred embodiment in the orosoluble solid oral composition for use according to the present invention, said S-adenosyl methionine is S-adenosyl methionine disulfate p-toluensulfonate.

Preferably said S-adenosyl methionine is contained in an amount from 50 to 500 mg, preferably from 100 to 400 mg, more preferably in an amount of about 200 mg (expressed in mg of SAMe ion).

According to a further preferred embodiment the orosoluble solid oral composition for use according to the present invention is in form of a tablet, capsule, powder, granule or granulate, preferably tablet or granulate, more preferably orosoluble granulate.

### Background

Mood disorders are psychopathological syndromes characterized by an alteration of the physiological mechanisms of mood tone oscillation, which normally allow the individual to adjust his relationships to the conditions of the surrounding environment. In mood disorders, the mood tone variations occur spontaneously, are of excessive extent and are accompanied by a wide range of symptoms, which lead the individual to being no longer capable of maintaining his normal daily functioning by completely altering his performances.

Anxiety is an innate component of human psychology which in normal conditions acts as a natural defense of man in particular situations; however, when anxiety becomes excessive and out of control, it connotes in an actual pathology with major complications and in comorbidity with other mood disorders.

Anxiety and depression are ever growing conditions in modem life and affect a significant part of the adult population, leading to a considerable distress which reflects on the life of both those affected subjects and any other person linked to them. Anxiety and depression, more and more often, lead people who suffer from it to have major relational difficulties, with limitations of social and interpersonal life which, in cases of greater severity and longer duration over time, become seriously limiting to social life. Depression can be primary or secondary (that is, present in patients already affected by other chronic diseases (diabetes, hypertension, for example) or acute diseases (cancer, TBC, HIV, for example). Recently, the *World Health Organization* (WHO) published an epidemiological study at the global level on the prevalence and incidence of depression and other common mental disorders such as anxiety. From the presented data, it is inferred that depression, besides being the major cause of suicide (800,000 dead/year), is the major cause of disability in the world. In Europe, depression affects 40.27 million people, whereas anxiety disorders affect 36.17 million people. *Mild depression* (mild or subthreshold depression) is a condition that is widespread among adult population, characterized by the same symptoms of major depression, although less incapacitating and less permanent over time (such as: depressed mood; cognitive alterations; nocturnal insomnia; difficulty in making decisions; loss of interest and pleasure; alterations of eating behaviour; physical symptoms; alterations of sexual behaviour; somatic complaints, such as fatigue, lack of energy, psychophysical malaise and sleep and appetite disorder; defects of concentration, affective disorders which present with sadness, lack of interest, dissatisfaction and detachment from pleasurable activities).

Anxiety and depression have been in the past, and unfortunately still today, considered "the evil of the century".

It is important to note how individuals affected by mood disorders and anxiety disorders normally have difficulties of both relational and interpersonal behaviour, based on the decrease of self-esteem, with little "willpower" for carrying out everyday habits and emotional balance deficit with repercussions on cognitive ability; this often leads to the erroneous or absent adherence to the assigned pharmacological and nutritional treatments. Adherence to treatment, or *compliance,* is crucial in order to obtain the desired benefit, especially on mood disorders.

To date, the available treatments often involve the intake of one or more products, even several times a day, maybe subdivided at precise times of the day, generating in the person further anxiety or even simple distress or nervousness. Adherence to treatment *(compliance)* is instead always a crucial aspect of each therapy, and in a greater measure for this type of treatments involving emotional sphere and psychological balance. It is known that patient adherence to antidepressant treatments is about 58% in the first month, and it decreases to 23% after 6 months. In case of secondary depression, that is indeed widespread among patients affected by chronic diseases (such as for example diabetes or hypertension), or acute diseases (such as for example cancer, TBC, HIV), depression has proven to be a limiting factor for patient adherence also to therapies for the main pathology. These patients are not objectively aware of having a low adherence to therapies, and this determines a deviation between the adherence subjectively perceived from the subject and the actual one. In case of secondary depression, therefore, the reduced compliance is not limited to the drugs for depression, but is extended to the drugs for the upstream pathologies and the effect of depression on the general *compliance* of the patient himself is therefore evident.

Drug therapy radically modified the evolution of chronic-degenerative diseases, including neuropsychiatric diseases, such as primary and secondary depression. Adherence, persistence, *engagement* (psycho-emotional involvement of the individual), *empowerment* (awareness and willpower of the patient himself as objective of his treatment) are fundamental concepts for a valid effectiveness of (poly)drug therapies. Many factors, relating to the patient, but also to the features of the product, to the health care system, to the doctor and to the environment, are at the base of scarce adherence. Patient adherence to the pharmacological treatment *(compliance)* is of fundamental importance for the success of therapeutic intervention in chronic diseases, including anxiety and depression. The problems linked to adhering to chronic drug therapy are always present, in the case of food supplements as well, when there is the need of daily administration.

The scarce adherence to therapeutic treatment of chronic diseases represents a widespread and important problem for many patients worldwide, resulting in a series of consequences, even serious, in the evolution of the pathologies for which these therapies have been prescribed. The term adherence refers to a behaviour through which patients comply with all of the doctor's indications and take drugs as prescribed by the doctor himself. Today, the doctor-patient relationship provides that the latter proactively collaborates to the implementation of the therapeutic project, instead of passively following the doctor's instructions. Conventionally, a threshold of 80% is used to categorize adherence to drugs as adequate or inadequate.

A second definition of compliance or persistence *("stay on therapy*") considers the time duration during which the patient takes a drug, even intermittently, before permanently discontinuing it.

Primary or secondary depression affects a significant amount of patients, sometimes with comorbidities, such as type 2 diabetes mellitus, often linked to mood disorder. The current pharmacology proposes a wide series of molecules useful for the treatment of depression, but usually adhesion to therapy is low and presents a series of variables and causal factors, with consequent non-achievement of therapeutic goals. A meta-analysis on 16 studies carried out worldwide, with 42,255 participants, detected that the non-adherence to drug therapy among patients with major depression disorders was as high as 50% (IC 95% 40%, 59%) (Semahegn et al. Systematic Reviews (2020) 9:17). Patient adherence to the antidepressant pharmacological treatment is an element of fundamental importance for the success of therapeutic intervention.

The SAMe (S-adenosyl-l-methionine), first discovered in 1952, is a fundamentally important molecule for our body and is present in all living cells. It is produced and activated in the liver, intervening as donor of methyl groups in a great number of biological reactions, which are catalyzed by enzymes of the methyltransferase family. The SAMe methyl group can be transferred by means of the action of such enzymes to oxygen, nitrogen or sulphur atoms present on other molecules, such as neurotransmitters, proteins and DNA.

A good and balanced nutrition constitutes the key factor for allowing to the body the necessary daily bioavailability of SAMe, estimated in about 500 mg and in plasma levels between 20 and 50 mg/ml (Bottiglieri T.: SAMe: from the bench to the beside-molecular basis of a pleiotropic molecule. Am. J. Clin. Nutr., 2002, 76:1151S-1157S).

The most known deficiency states are represented, for example, by senescence, wherein a decrease in SAMe greater than 50% is described, from the neurological affections which involve the affective sphere (depressive syndromes in general) and cognitive sphere (dementia), from hepatic disorders, both acute and chronic (alcoholic steatosis, acute and chronic hepatitis, alcoholic and postviral cirrhosis, intrahepatic cholestasis) and from musculoskeletal degenerative diseases characterized by alterations in mobility (osteoarthritis) (Grazi S., Costa M. : SAMe: The safe and natural way to combat depression and relieve the pain of osteoarthiritis. Prima Health, Rocklin, CA, 1999).

The idea of exogenous SAMe administration to patients was pursued ever since the discovery of the molecule, in the fifties, but it became possible only starting from the second half of the seventies, when the first parenteral (intravenous and intramuscular) or oral (gastro-protected or rather gastro-resistant swallowable tablets) preparations of SAMe salts (such as for example tosylate, butadienesulfonate or phytate), stable at room temperature, were introduced in the pharmaceutical market.

It is known that the SAMe molecule is very unstable at temperatures higher than 0°C. Besides thermolability, the second important degradability factor of the molecule is represented by its hygroscopicity which can be remedied by conserving the thermostable SAMe salts in designated protective enclosures or by lyophilizing the salt under vacuum and putting it into vials. It is therefore known the technical processing difficulty of the molecule, especially at the level of industrial stocking and manipulation. SAMe is a very unstable molecule.

It is further known that, while the exogeneous SAMe administration via parenteral (i.e., intravenous or intramuscular) route ensures high levels of plasma bioavailability (corresponding to about 90-100% of the administered dose), oral intake of SAMe produces modest plasma levels of the unmodified molecule, i.e., < 5-10% of the administered dose.

This significant bioavailability gap derives from the high presystemic metabolism at the hepatic level of orally ingested SAMe. The metabolic activity of the liver for SAMe drastically reduces the systemic bioavailability levels of the molecule. It is therefore necessary to be able to process and formulate SAMe adequately in order to make it bioavailable, even when formulated for oral administration.

In addition to the above, SAMe is also characterized by an unpleasant odor and taste, which are misperceived by subjects who must take it. Odor and taste of the active substance may influence in a very significant way the final formulation of the product and the consequent acceptance and successful continuation of the treatment by the patient.

Odor and taste are important organoleptic features of the product and of its components and therefore may contribute to adherence to therapy *(compliance)* and subsequently to the success of the therapy. The importance of therapeutic adherence represents a constant challenge of the world of pharmaceutical and food industry to improve the *compliance* of products, the facility of intake and the "taste" of finished products. Over the years stable pharmaceutical formulations have been developed, which favour the absorption of the SAMe molecule and also ensure good palatability in the case of orosoluble pharmaceutical forms. The European patent EP2393475B1 and the application of Italian 102019000015192 describe some of these. Despite the efforts that technology has made thus far, SAMe remains an active substance which is extremely complex to process, due to its instability, hygroscopicity, limits of bioavailability when formulated for oral administration and, not last nor less important, the unpleasant odor and taste.

In the central nervous system, many important reactions are determined by the presence of SAMe; among these, the synthesis of various neurotransmitter monoamines, such as adrenaline, dopamine, serotonin, and histamine. SAMe intervenes, therefore, in the biosynthesis of hormones linked to mood, allowing the improvement of information transmission between cells of the nervous system with a positive effect on mood itself.

It is therefore known that SAMe has an effect on modulating mood tone and in the antidepressant treatment. Existing literature describes the antidepressant activity of SAMe as being comparable to that of two important reference drugs (clomipramine and imipramine) or to that of synthetic tricyclic antidepressants. Considering also that the majority of treatments for these disorders is associated with side effects, scarce or no compliance/adherence to the treatment by the patient, these data prove that SAMe, a naturally molecule present in our body and on the contrary almost lacking side effects, is a compound of remarkable interest for the treatment of patients with mood depression, in particular if accompanied by somatic concomitant comorbidities.

The human gastrointestinal microbiota consists of ingested and resident microorganisms, it is essential to human health and contributes to the digestion of food and optimal functioning of the immune system (FAO, 2001). Microorganisms, recognized as probiotics, belong to *Lactobacillus o Bifidobacterium.* Probiotics (or lactic ferments) are living and active microorganisms (especially bacteria), which can be administered in a sufficient number so that to exert a positive effect on the body health, strengthening in particular the intestinal ecosystem.

Traditionally, clinical application of probiotics includes the prevention and treatment of gastrointestinal infections. The mechanisms of action by which probiotics may contribute to human health are grouped in three categories; the first relates to the ability of probiotics to exclude or inhibit pathogens, and this is the best-studied mechanism in literature. A further mechanism is the activation of the function of the intestinal barrier with the modulation of pathways which lead to mucus secretion, to the activation of tight junctions between intestinal cells (Seth A. et al. Probiotics ameliorate the hydrogen peroxide-induced epithelial barrier disruption by a PKC- and MAP kinase dependent mechanism. Am. J. Physiol. Gastrointest. Liver Physiol. 2008. 294: G1060-G1069) and to apoptosis prevention. Furthermore, probiotics can modulate the host immune response with both a local and systemic action. Besides intestinal epithelial cells, probiotics may come into contact with intestinal dendritic cells which have a crucial role in innate and adaptive immunity.

By virtue of the benefits brought to the host, bifidobacteria are considered one of the most important probiotic microbial groups. Their probiotic effect is due to several mechanisms. One of the main beneficial effects of bifidobacteria consists in lowering the pH of the ascending tract of the colon. Indeed, the metabolism of carbohydrates in *Bifidobacterium* mainly produces acetic acid and lactic acid which inhibit the development of many potentially pathogenic acid-sensitive microbial species by favouring their elimination. *Salmonella, Shigella, Clostridium, Bacillus cereus, Staphilococcus aureus* and *Candida albicans* are among the pathogens that are sensitive to the inhibitory action of bifidobacteria. A further benefit brought by some species of bifidobacteria consists in the secretion of aminoacids and vitamins which will be available for use by the intestinal microbiota or the host. Notably, the production of folic acid by some *Bifidobacterium* strains results particularly useful in preventing the onset of neoplastic forms of colonocytes.

One of the main mechanisms through which probiotics may positively influence the health of the host is based on their ability to interact with the host immune system by eliciting responses both at the local level and the systemic level. Several studies reported the ability of strains belonging to the *L. helveticus* species to exert immunostimulating effects, both alone and in combination with other bacterial strains. For example, the *L. helveticus* R0052 strain was used in combination with *B. longum* to ferment both a soya-based drink and a milk-based mixture. When these mixtures were added to HT29 and T84 intestinal epithelial cell lines before stimulation with tumor necrosis factor (TNF)-a, they significantly reduced the expression of interleukin (IL)-8 chemokines.

The administration of milk fermented from *L. helveticus* strains represents a potential alternative treatment for the prevention of enteric infections, probably mediated by the production of bioactive compounds. It has been demonstrated that peptides derived from the high proteolytic activity of *L. helveticus* on food proteins stimulate the host immune system. Probiotics, beside intervening on immune responses, also intervene on some aspects of the functionality of our brain; as a matter of fact, a gut-brain axis exists which suggests the potentiality of employing probiotics in the prevention and control of specific mental illnesses. Brain and intestine closely communicate with each other through a thick neural network of 500 million neurons which innervate the intestine. This vast connection network monitors the whole digestive tract from the esophagus to the anus. Although capable of performing its functions completely autonomously with respect to the central nervous system, the enteric nervous system is closely interconnected thereto, by creating a complex morpho-functional axis through which such systems communicate and influence each other (gut/brain axis). The central nervous system is capable of modifying the intestinal function through the endocrine system (hypothalamic-pituitary-adrenal axis) and the autonomous nervous system. More recently, it emerged that the nervous central system is further capable of modifying the composition of the bacterial flora and to regulate intestinal permeability. WO2010//089674A1 discloses an orosoluble composition, e.g. a tablet, capsule or granule, containing a salt of S-adenosyl methionine (SAMe), such as the tosylate, and a further active ingredient, which can be a probiotic, such as bifidum bacterium longum, or a lactobacillus, and its use in the treatment of depression or anxiety. Example 10 discloses an orosoluble tablet containing SAMe butanedisulfonate (127.8 mg SAMe), Lactobacillus acidophilus and Bifidum bacterium longum. The orosoluble composition can contain also vitamin B6.

WO2016/065419A1 describes the treatment or prevention of anxiety or depression, that can be mild or post- partum depression, with a composition, e.g. a capsule, comprising orotic acid and S-adenosyl methionine (SAMe), a probiotic and CoQ. The probiotic can be Bifidobacterium longum or Lactobacillus. One or more of the strains may be encapsulated in, for example, a suitable polymeric matrix, such as alginate beads, to improve long term stability and storage of the compositions. SAMe can be SAMe disulfate monotosylate and MgO may be an optional carrier. Michael Messaoidi et al. (BRITISH JOURNAL OF NUTRITION, vol. 105, no. 05, 26 October 2010 (2010-10-26), pages 755-764) teaches that L. helveticus R0052 and B. longum R0175 taken in combination (PF) display anxiolytic-like activity in rats and beneficial psychological effects in healthy human volunteers. PF appears to show a beneficial effect on general signs of anxiety and depression.

Recent evidence shed light on the possibility that the enteric nervous system and the microbiota may in turn modify the cerebral development and functions. In particular, the microbiota would have a central role in the production of neurotransmitters or precursors thereof such as, for example, aminoacids (GABA5, Tryptophan) and monoamines (Serotonin, Histamine).

S-adenosyl methionine and probiotics have thus a role at the level of the gut-brain axis.

As underlined above, it is known that the S-adenosyl methionine (SAMe) molecule is very complex to process, and brings along numerous technical difficulties making complex to arrive at the stable final product which has an acceptable taste, in particular when formulated for an oral administration.

Probiotics are not exempt from processing difficulties as well. It is in fact well known the difficulty of formulating compositions containing probiotics which are able to reach alive and active at the site of action. Probiotics are very delicate substances or living organisms, which can easily be inactivated by their intrinsic fragility as well as by interaction with other substances required for the formulation such as, for example, solvents or diluents and by exposure to temperature changes, excess of humidity or other stimuli.

It is therefore clear how the technical difficulty linked to SAMe, along with the features of complexity on the preparation of products containing alive probiotics discourages the average technician of the field to evaluate a combination of the two, even more if for oral administration in form of solid composition, which is stable at room temperature.

In this regard it should be noted how, from the therapeutic point of view, in order to act at the level of the gut-brain axis it is indispensable that the SAMe is released quickly and effectively to be as bioavailable as possible, while it is necessary that probiotics resist the gastric passage to arrive alive and active in the intestine, where they perform their activity.

To date, there is no single composition comprising the claimed combination of SAMe and/or salts thereof and mixture of probiotics, both present in a single composition.

To date, there are no available products capable of joining together in a single composition these substances such as to obtain a synergistic effect without side effects and maintaining such components stable. It should be also noted, in fact, that the majority of the currently used products for the treatment of several mood disorders presents important side effects, which determine a very controlled, if not limited use thereof. Also, a similar aspect has a certain relevance on the *compliance.* It is therefore felt the need to overcome the technical difficulties linked to each of the substances above, both SAMe and probiotics, even more if formulated together in a single composition for oral administration.

It is furthermore felt the need to find new effective solutions with no side effects for the treatment and/or prevention of mood disorders, notably anxiety and primary or secondary depression (both generalized Anxiety Disorder, Minor Depression, reactive Depression and Unipolar Major Depression) and all the symptoms correlated thereto.

As indicated above, secondary depression, which is a widespread condition among patients affected by chronic diseases (for example diabetes or hypertension), or acute diseases (cancer, TBC, HIV), may bring along the failure to adhere not only to the depression treatment but also to the treatment of the main pathology.

### Description

Surprisingly, it has now been found that a new composition according to claim 1 comprising the combination of S-adenosyl-l-methionine (SAMe and/or salts thereof) with a mixture of probiotics consisting of Lactobacillus helveticus Rosell^{®}-52 and Bifidobacterium longum Rosell^{®}-175, specifically formulated in orosoluble solid oral form, provides a simultaneous supplementation of these components without nullifying the stability of the two components. Thanks to this new formulation it is now possible to obtain one or more of the following advantages at the level of processing/production, of finished product and/or at the level of treatment, and that is i) overcoming the technical difficulties linked to the formulation of these substances singularly and even more in combination with each other; ii) the obtainment of a product containing stable, palatable, highly bioavailable SAMe; iii) the obtainment of a product containing alive probiotics, capable of arriving unaltered to the intestine; iv) the maintenance of probiotics in active form, without degradation; it is in fact surprising how, despite the acidity of SAMe, there is no degradation of probiotics thanks to the new formulation of the invention; v) an integrated effect of substances formulated together in a single composition; vi) an improved therapeutical adherence *(compliance)* by the patient and vii) a synergistic effect between SAMe and said at least one probiotic. Such advantages eventually lead to an improved efficacy on mood disorders, with no side effects.

An object of the present invention is therefore an orosoluble solid oral composition comprising the combination of S-adenosyl methionine with a mixture of probiotics consisting of Lactobacillus helveticus Rosell^{®}-52 and Bifidobacterium longum Rosell^{®}-175, in which said probiotics are present in the combination in a microencapsulated form, at least one physiologically acceptable excipient and an additional agent selected from a mixture of magnesium oxide and vitamin B6. Further object of the present invention is a composition according to the present invention, for use in the treatment and/or prevention of mood disorders, preferably for the treatment of anxiety, depression, psychological stress, mild postpartum syndromes and/or stress related instinal disorders.

Further disclosed is a solid oral composition, preferably orosoluble, comprising S-adenosyl methionine (SAMe) in combination with at least one probiotic and at least one physiologically acceptable excipient where said SAMe is in stable and/or palatable form.

Further disclosed is a solid oral composition, preferably orosoluble, comprising S-adenosyl methionine (SAMe) in combination with at least one probiotic and at least one physiologically acceptable excipient, where said at least one probiotic is alive and active, and reaches the intestine in an alive and active form.

Further disclosed is a solid oral composition, preferably orosoluble, comprising S-adenosyl methionine (SAMe) in combination with at least one probiotic and at least one physiologically acceptable excipient where said composition improves patient therapeutical adherence to the treatment *(compliance).*

Further disclosed is a solid oral composition, preferably orosoluble, comprising S-adenosyl methionine (SAMe) in combination with at least one probiotic and at least one physiologically acceptable excipient where said SAMe and at least one probiotic remain stable in the composition.

Unless otherwise specified, within the scope of the present invention a range of values indicated for a quantity, for example the weight or percentage content of a component, includes the lower limit and the upper limit of the range. For example, if the weight or volume content of a component A is indicated as "from X to Y", where X and Y are numerical values, A may be X or Y or anyone of the intermediate values.

Unless otherwise specified, within the scope of the present invention the indication that a composition "comprises" one or more components or substances means that other components or substances may be present beside that, or those, specifically indicated. The terms "comprising", "having", "including" and "containing" are to be intended as open terms (i.e., meaning "comprising, but not limited to").

Unless otherwise indicated, within the scope of the present invention the term "physiologically acceptable excipient" refers to a substance lacking in any pharmacological effect of its own and which does not produce adverse reactions when administered to a mammal, preferably a human being. Physiologically accepted excipients are well known in the art and are described, for example, in the Handbook of Pharmaceutical Excipients.

Unless otherwise indicated, within the scope of the present invention the term "simultaneous" refers to the contemporaneous administration of active substances, in the same composition, such that the compounds act together in the body, giving origin to a contemporaneous integration thereof.

Unless otherwise indicated, within the scope of the present disclosure the term S-adenosyl methionine refers to the S-adenosyl methionine (or S-adenosyl-l-methionine or SAMe) molecule and/or to anyone of the pharmaceutical acceptable salts thereof.

Preferably, S-adenosyl-methionine salt is S-adenosyl methionine disulfate p-toluensulfate.

Said at least one probiotic according to the present disclosure preferably belongs to the *Lactobacillus and*/*or Bifidobacterium* class and is more preferably selected from *Lactobacillus helveticus, Bifidobacterium longum* or a mixture thereof.

The *Lactobacillus helveticus* Rosell^{®}-52 strain belongs to the genre of Lactobacilli, it was isolated in 1990 and its genomic sequence was entirely mapped and it was deposited in France with the identification code I-1722-CNCM. In accordance with the present invention, it is important to note that said strain is capable of surviving well along the gastrointestinal tract, adhering to the intestinal epithelium cells, displacing pathogens and increasing the barrier function.

The *Lactobacillus helveticus Rosell*^{®}-*175* strain belongs to the genre of Bifidobacteria, it was isolated in 1988 and its genomic sequence was entirely mapped and it was deposited in France with the code I-3470-CNCM. In accordance with the present invention, it is also important to note here that such strain survives well in the gastrointestinal tract and carries out an antimicrobial activity. Such probiotic strain proves to be active against a great number of intestinal pathogens (such as, for example, *E.Coli, C. difficile, S. aureus)* and is capable of reducing the production of inflammatory cytokines, therefore positively modulating the immune response and decreasing intestinal permeability.

Unless otherwise specified the term "orosoluble" according to the present invention refers to compositions capable of dissolving and releasing immediately the active substance contained therein, when in contact with the oral mucosa. In this way the active substance can be directly absorbed in the oral mucosa, thus avoiding the hepatic circle.

The term orosoluble according the invention is therefore aimed at compositions to insert in the oral cavity, where the active substance S-adenosyl methionine becomes immediately orodispersible and available.

Thanks to this specific formulation the S-adenosyl methionine is absorbed right away, ensuring an improved bioavailability thereof. According to a preferred embodiment, in fact, the absorption is ensured by the orosoluble form, which allows to solve the problems of scarce bioavailability which generally characterize the oral forms of this substance.

The term "oral" according to the present invention refers to a composition aimed at administration by mouth. The oral composition according to the invention is in orosoluble solid form, . Preferably, said solid oral form of the invention is in form of a tablet, capsule, powder, granule or granulate, preferably granulate or tablet, more preferably orosoluble granulate.

According to the disclosure, the solid oral form of the invention is orosoluble tablet or modified-release multilayer swallowable tablet.

According to the disclosure the solid oral form of the invention is effervescent orosoluble and/or soluble granulate.

According to the disclosure, the composition according to the present disclosure is packaged in sachet, preferably single-dose sachet or bipartite sachet.

According to the disclosure the composition of the present disclosure is in form of an orosoluble granulate, packaged in single-dose sachet (*stick pack*)*.*

The probiotic strains are present in the composition according to the present disclosure in a preferably gastro-resistant form, such to go beyond the gastric district and to reach the intestine where they must colonize and perform their action. More preferably, probiotic strains are present in the composition in microencapsulated form. The microencapsulated form according to the invention ensures the probiotic strains a greater stability, such to allow going beyond the stomach and reaching the intestine in alive and active form. Thanks to the microencapsulation the probiotics of the invention are therefore gastro-resistant, stable from the moment of intake up to the site of action.

As said above, the probiotics are very delicate substances or living organisms and going beyond the stomach, with the reaching of the intestine in alive and active form represents one of the formulation hurdles to overcome. Gastro-resistance becomes a crucial aspect in the processing of the probiotic, as preferably comprised in the composition of the disclosure. Thanks to their new microencapsulated form it is possible to obtain such result. Thanks to this specific composition, probiotic strains arrive to the intestine alive and active, ensuring the best efficacy.

Said at least one physiologically acceptable excipient according to the present disclosure can be selected from diluents, aggregants or binders, lubricants, glidants, disaggregants, solubilizers, sweeteners, flavoring agents, pH adjusters, or a mixture thereof.

The composition according to the present disclosure may further comprise one or more additional ingredients, preferably selected from minerals and/or vitamins. More preferably, said minerals are chosen from magnesium, potassium, calcium, phosphorus, iron, zinc, copper, manganese, fluorine, selenium, chromium, molybdenum, iodine, boron, chlorine, sodium and silicon or other mineral comprised in the corresponding Table of the Italian Ministry of Health or a mixture thereof, whereas said vitamins are vitamins group B, preferably selected from Vitamin B1, Vitamin B6, Vitamin B12, or Vitamin B9 or other vitamin such as Vitamin A, Vitamin D, Vitamin E, Vitamin K, Vitamin C, riboflavin (Vitamin B2), Niacin, Folic Acid, Biotin, Pantothenic Acid, or other Vitamin comprised in the corresponding Table of the Italian Ministry of Health or a mixture thereof. Magnesium oxide and vitamin B6 contribute synergistically to rebalance the nervous system and normalize the psychological function and are present in the claimed orosoluble solid oral composition.

The synergistic effect given by the composition of the invention is further amplified by the combined effect of magnesium oxide and vitamin B6, which flanks the integrated effect of the simultaneous administration of SAMe and probiotics.

The S-adenosyl methionine (SAMe) is contained in the composition of the present invention in an amount that varies from 50 mg to 500 mg, preferably from 100 mg to 400 mg. More preferably, the S-adenosyl methionine is contained in the composition of the present invention in an amount of about 200 mg. The amounts of SAMe in the composition are here expressed as mg of SAMe ion; the amounts here expressed are intended for single composition, preferably expressed in mg for sachet (mg/*stick pack*), always expressed as mg of SAMe ion.

Said at least one probiotic is contained in the composition of the present disclosure in an amount that varies from 0.5 x 10⁹ CFU and 30 x 10⁹ CFU (Colony-Forming Units), preferably from 1 x 10⁹ CFU to 5 x 10⁹ CFU. More preferably said at least one probiotic is contained in the composition of the present disclosure in an amount of about 3 x 10⁹ CFU. According to a preferred disclosure, the probiotic is represented by the one mixture of probiotics which preferably corresponds to a *Lactobacillus* and *Bifidobacterium* mixture, more preferably as discussed above a *Lactobacillus helveticus* and *Bifidobacterium longum* mixture. Therefore, the probiotic mixture is contained in the composition of the present disclosure in an amount that varies from 0.5 x 10⁹ CFU and 30 x 10⁹ CFU (Colony-Forming Units), preferably from 1 x 10⁹ CFU to 5 x 10⁹ CFU. More preferably said at least one probiotic is contained in the composition of the present disclosure in an amount of about 3 x 10⁹ CFU. The amounts here expressed are intended for single composition, preferably expressed in sachet (*stick pack*), or for daily dose.

The vitamin group B is present in the composition of the present disclosure in an amount that varies from 5% VNR to 1250% VNR (Value Nutritional Rate), preferably from 15% to 300% VNR. More preferably said vitamin is contained in an amount of about 100% VNR. According to a preferred embodiment of the present invention said vitamin is vitamin B6 (Pyridoxine Hydrochloride powder). The mineral is contained in the composition of the present disclosure in an amount that varies from 5% VNR to 300% VNR (Value Nutritional Rate), preferably from 10% VNR to 100% VNR. More preferably, said mineral is contained in an amount of about 15% VNR. According to the description the mineral is magnesium oxide, the dosage of which corresponds to a daily dose of magnesium of about 15% VNR. The amounts here expressed are intended for single composition, preferably expressed in sachet (*stick pack*)*,* or for daily dose. The composition according to the present invention is indicated for use in the treatment and/or prevention Minor or Subthreshold Depression (*mild-depression*).

With the term "mood disorders" according to the present invention, a wide series of disorders and/or pathologies which involve the central nervous system, with respect to the emotional and psychological balance, are meant to be taken into account. In particular, the composition of the present disclosure is useful in case of anxiety, depression, psychological stress (of various origin such as, for example, work, family, study or other), mild *post-partum* syndromes (such as *post-partum* Blues Syndrome) and/or intestinal disorders correlated to stress (such as for example irritable bowel syndrome, leaky gut syndrome) and burn-out symptoms. The depression can be primary depression or secondary depression, where for secondary a widespread condition among many patients already affected by chronic diseases (for example diabetes or hypertension) or acute diseases (for example cancer, TBC, HIV) is intended.

The depression, whether minor or subthreshold, can also be correlated to changes of the hormonal component, in particular for women during the transition phase toward menopause (perimenopause) or during menopause itself. Menopause and depression can also be an association altering the optimal functioning of many women. It is an evidence base medicine datum that the risk of depression increases during perimenopause, in case of early menopause and in the period after menopause. The correlation between menopause and depression appears to be closely linked to hormonal changes which characterize this phase of a woman's life. At the same time, however, the stress and culture with which this physiological change is faced and experienced can also affect our mood, even going so far as to affect the subtle biochemical balances that regulate it.

"Minor" depression in menopause is therefore an eventuality to evaluate with care and awareness to prevent and treat a physical and mental problem which can limit a woman's life and which represents an important risk factor for her health. The composition of the invention can be particularly advantageous in these cases of hormonal imbalance, with also an improvement of the dysbiosis with consequent attenuation of inflammatory and degenerative phenomena of the mucous membranes.

According to the disclosure, the composition is administered with a posology of one single-dose sachet (*stick-pack*) per day, preferably one sachet in the morning after the first main meal. More preferably for at least two weeks.

The single daily administration of the composition of the disclosure is correlated to an improved acceptance of the treatment by the patient who in this way overcomes the possible distress of multiple intakes *(compliance).* Having to take different compositions or products, possibly several times a day and/or at different times of the day, is one of the problems often encountered in the patient affected by emotional imbalance and mood disorder. It is important that the patient adheres in a precise and continuous way to the treatment, and the administration of a single composition, preferably once a day, solves the problem. This datum is based on EBM: in chronic therapies, the patient who already has to take multiple drugs a day prefers the single administration. The patient's subjective perception of taking numerous ("too many" or as such perceived) drugs often leads the patient to a reduced *compliance.* The fixed combination of active substances and/or ingredients according to the present invention, with respect to the current individual administrations of multiple products, even several times a day, leads to the advantage of an improved *compliance.*

Such type of composition, administration and/or packaging of the product therefore improves *compliance* of the patient, to whom, thanks to the composition of the invention, the necessary intake of both SAMe and probiotics is provided, optionally with the addition of one or more further ingredients. As already said above, patient adherence to the pharmacological treatment *(compliance)* is of fundamental importance for the success of therapeutic intervention in chronic diseases, including anxiety and depression (both primary and secondary).

The present composition in form of orosoluble granulate is packaged in single-dose sachet (*stick-pack*).

### Experimental Part

### 1.COMPATIBILITY STUDY

A compatibility study intended to demonstrate the possibility to mix SAMe (or its salt) and probiotics was executed. The probiotics to be tested have been selected in accordance with the composition of the invention such as *Lactobacillus helveticus Rosell*^{®}*-52, Bifidobacterium longum Rosell*^{®}*-175* and a mixture of the two. The study was further conducted using such probiotics in both not microencapsulated and microencapsulated forms.

The study was carried out in a first phase with SAMe *+ Lactobacillus helveticus R0052,* not microencapsulated, through a liquid compatibility method. The initial population tested was 15.10^{E}9 CFU/g units at 500mg of SAMe. For this evaluation a pHmeter with 8 probes was used, and values were verified every 15 minutes. The strains are inoculated into a liquid medium suitable for their growth, only under aerobic conditions (reason why in this way the *Bifidobacterium* is not tested), and the active substance is inoculated into the sample and not into the control. The compatibility evaluation was executed monitoring the pH, as following:
- if an acidification is found: a multiplication of the strain (natural fermentation) occurs and therefore no negative interaction with the active substance is produced;
- if a light acidification of the medium is found: a multiplication of the strain occurs, but slower than it should be; the method of analysis must be adequate to the time of the microorganisms count on the finished product;
- if no acidification is found: the active substance shows bacteriostatic or lethal effect on the strain.

From an operational point of view: the vial is filled with the liquid medium (50 ml RCM); the active, which should have been tested directly in its granulated form in the vial, is added, and it is mixed. Then the pH of the medium in the presence of the active is verified, if necessary, the pH is adjusted so that it is comprised between 6.5 and 7. R0052 strain is therefore inoculated (batch U12171600, 500.10^{E}9 CFU/g) into the vial. The sample is diluted 1/100 with Dilumat to obtain a dilution to 5.10^{E}9 CFU/ml and finally it is inoculated with 3 ml into the 50 ml RCM vial, obtaining 15.10^{E}9 CFU/ml in vial. The vial is placed in warm bath, at 37°, and the pH started to be recorded for 18 hours.

**Conclusion:** it was seen that pH of the R0052 strain decreases from 6.1 to 4.2: it is the natural multiplication of the bacteria that acidifies the medium. The pH of the active (SAMe) remains essentially unchanged, stable and it is neutral (7-7.5). When R0052 is put in contact with the SAMe sample, the pH decreases from 7.2 to 4.2. Consequently, it is demonstrated that the L. *helveticus* R0052 strain appears compatible with the SAMe.

1.2 MICROBIOLOGICAL TEST: within the bio-compatibility study a microbiological evaluation was carried out. R0052 and R0175 probiotic strains were cultured in a Petri dish, in the presence of the active (SAMe, marketed formulation) at a 500 mg dose (as above). A sample was also analyzed as a reference of probiotic strain only, so that it could be compared with the addition of the active on the result in Colony-Forming Units (CFU). The obtained results showed that the insertion of the SAMe in the presence of each probiotic strain (R0052 o R0175) modifies the CFU count only up to 5%. This is easily justified by the simple analytical error, known in the microbiological field. These results therefore confirm what already seen with the compatibility test, demonstrating again that the probiotic strains are compatible with SAMe.

*2.SAMe - MIVO*^{®} *STUDY:* in vitro study of absorption of S- Adenosil-L-Metionina (SAMe), through the buccal epithelial tissue and MIVO^{®} device.

The in vitro study of absorption was carried on with the utilization of reconstructed oral epithelial tissue Reconstructed Human Oral Epithelium (HOESS kit of 12 tissues) of EpiSkin Company mounted on the MIVO^{®} Bulk2 Box (12 MIVO^{®}) device of React4life Company.

Given the orosoluble characteristic of the composition as described below in Example 1, in a first experimental phase the oral digestion of the active is mimicked through incubation of the samples with solutions of artificial saliva.

Subsequently to the first digestive phase, the assay of the absorption of SAMe through HOES5 follows, a 3D model of epithelial tissue free of the stratum corneum, which histologically resembles the mucosa of the oral cavity, usable for tests of toxicity, metabolic, absorption and efficacy of the compounds.

The absorption assay is executed in triplicate mounting the *transwell* of the HOES5 tissue on the MIVO^{®} device, which combines 3D tissues and fluidic circulation, reproducing physiological conditions for the tissues at the following experimental timepoints: T10 (10 minutes after the insertion of the digested sample in contact with HOES5 tissue), T20 (20 minutes after the insertion) and T30 (30 minutes after the insertion).

Subsequently, the quantitative analysis of the analyte SAMe ion, present in the formulation of the Example 1 pre-digested, digested and after each experimental timepoint, is carried out through RP-HPLC chromatographic analytical technique, including fluorometric and UV detector.

Each sample is analyzed at a non-toxic concentration for the cells of the buccal tissue, the same is derived starting from the present SAMe quantity and from the posology of the finished product.

The permeability of the tissue to each active is expressed in terms of percentage of permeation with respect to the applied quantity.

All the experiments are carried out in triplicate (biological replicates) and the results expressed as mean ± SEM (standard error of the mean). The statistical significance between the groups is evaluated through two-tailed Student t test (p < 0,05), after evaluation of the normal distribution of the data.

The expected results for such *in vitro* study demonstrate the absorption of the SAMe according to the invention through the buccal epithelial tissue.

### 3.CLINICAL EFFICACY STUDY

A study on the efficacy of the composition according to the present invention is under evaluation, as reported in example 1, in patients with minor or subthreshold depression (*mild-depression*): the clinical study is single-center, controlled, randomized, parallel-group, cross-over, with *run-in* and *follow-up* periods, double-blind vs placebo.

Primary objective of the clinical study is to evaluate the efficacy of the composition of the present invention as food supplement based on S-adenosyl methionine (SAMe), *Lactobacillus helveticus* and *Bifidobacterium longum* in improving and/or preventing the mood tone disorders.

Such evaluation will be measured through the administration to the enrolled subjects of validated questionnaires:
- Hamilton Depression Rating Scale - HAM-D (administered by the doctor),
- General Health Questionnaire - GHQ-1 (self-administered in the doctor's office).

Perceived Stress Scale - PSS Test (self-administered in the doctor's office). Secondary objective of the study is to evaluate the efficacy of the composition of the present invention as food supplement based on S-adenosyl methionine (SAMe), *Lactobacillus helveticus* and *Bifidobacterium longum* in cases of anxiety disorders. Such evaluation will be measured through the administration to the enrolled subjects of the validate questionnaire:
- Hamilton Anxiety Rating Scale (HAM-A) (administered by the doctor); and
- Self-administered tests to carefully evaluate the psychic condition of the investigator.

The experimental groups will be the following:
Group 1: subjects who will take the treatment daily; and
Group 2: subjects who will take the placebo daily.

The total duration of the study will be 12 months: 1 month for patients enrollment, 3 months of treatment, 1 month of wash-out, a cross-over design and finally 6 weeks of follow-up.

### 4. STABILITY STUDY

The stability of the composition as described in example 1 has been evaluated for a period of 5 days in the oven at 50°. Control parameters were:
- Appearance
- Mean weight
- % H2O determined through KF (Karl Fischer) method
- Assay of SAMe active
- Degradation products

The product showed to be stable for 5 days, at 50° temperature, as it can be inferred from the table below. Such accelerated stability test is an industry standard with a well-established use for products based on SAMe.

| | **SAMe + PROBIOTICS BATCH: 1518-7** | |
|---|---|---|
| ANALYSIS | TIME ZERO | 5 DAYS |
| **APPEARANCE** | White powder | Unchanged |
| **MEAN WEIGHT** | 1.889 g/bst | 1.886 g/bst |
| **K.F.** | 0.732% | 0.744% |
| **ASSAY** | 187.96 mg/bst | 169.74 mg/bst |
| **DEGRAD. PROD.** | 1.46% A (0.04 W/W) | 3.88% A (0.09 W/W) |

The following examples are included herein to better describe the present invention, without limiting it in any way.

### EXAMPLES

| **EXAMPLE N1** | |
|---|---|
| PHARMACEUTICAL FORM | **OROSOLUBLE GRANULATE IN STICK PACK** |
| | |

| INGREDIENTS | mg/stick pack |
|---|---|
| S-adenosyl-L-methionine disulfate p-toluensulfonate (SAMe) | **400** |
| of which SAMe ion | 200 |
| Mixture of lactic ferments (Lactobacillus helveticus Rosell-52, Bifidobacterium longum Rosell -175) | 3x10⁹ CFU |
| Magnesium oxide | **93.3** |
| of which magnesium | 56.25 |
| Vitamin B6 (Pyridoxine Hydrochloride) powder | 1.7 |
| of which Vitamin B6 | 1.4 |
| Xylitol | **660.68** |
| L-Arginine | **150** |
| Mannitol | **100** |
| Fatty acids | **80** |
| Flavour | **18.79** |
| Silicon dioxide | **9.15** |
| Sucralose | **1.37** |
| Acesulfame k | **0.85** |
| Neohesperidine DC | **0.16** |
| Total per stick pack | **1900** |

| **EXAMPLE N2** | |
|---|---|
| PHARMACEUTICAL FORM | **OROSOLUBLE GRANULATE IN SACHET** |
| | |

| INGREDIENTS | mg/sachet |
|---|---|
| S-adenosyl-L-methionine disulfate p-toluensulfonate (SAMe) | 500 |
| of which SAMe ion | 250 |
| Mixture of lactic ferments (Lactobacillus helveticus Rosell-52, Bifidobacterium longum Rosell -175) | 6x10⁹ CFU |
| Magnesium oxide | **186.6** |
| of which magnesium | 112.5 |
| Vitamin B6 (Pyridoxine Hydrochloride) powder | **11.53** |
| of which Vitamin B6 | 9.5 |
| Xylitol | **1237.37** |
| L-Arginine | **200** |
| Mannitol | **1000** |
| Fatty acids | **50** |
| Flavour | **25** |
| Silicon dioxide | **15** |
| Sucralose | **5** |
| Acesulfame k | **1** |
| Neohesperidine DC | **0.5** |
| Total per sachet | **4000** |

| **EXAMPLE N3** | |
|---|---|
| PHARMACEUTICAL FORM | **MODIFIED-RELEASE MULTILAYER SWALLOWABLE TABLET** |
| | |

| INGREDIENTS | mg/tbl |
|---|---|
| S-adenosyl-L-methionine disulfate p-toluensulfonate (SAMe) | **400** |
| of which SAMe ion | 200 |
| Mixture of lactic ferments (Lactobacillus helveticus Rosell-52, Bifidobacterium longum Rosell -175) | 3x10⁹ CFU |
| Magnesium oxide | **93.3** |
| of which magnesium | 56.25 |
| Vitamin B6 (Pyridoxine Hydrochloride) powder | 1.7 |
| of which Vitamin B6 | 1.4 |
| Maltodextrin | **116** |
| Microcrystalline cellulose | **100** |
| Fatty acids | **80** |
| red iron oxide | **5** |
| Silicon dioxide | **10** |
| Magnesium stearate | **5** |
| Ethylcellulose | **5** |
| Total per tablet | **1200** |

| **EXAMPLE N4** | |
|---|---|
| PHARMACEUTICAL FORM | **OROSOLUBLE TABLET** |
| | |

| INGREDIENTS | mg/tbls |
|---|---|
| S-adenosyl-L-methionine disulfate p-toluensulfonate (SAMe) | 250 |
| of which SAMe ion | 125 |
| Mixture of lactic ferments (Lactobacillus helveticus Rosell-52, Bifidobacterium longum Rosell -175) | 1.5x10⁹ CFU |
| Magnesium oxide | **93.3** |
| of which magnesium | 56.25 |
| Vitamin B1 | **2.5** |
| Vitamin B2 | **2.5** |
| Vitamin C | **80** |
| Xylitol | **717.19** |
| L-Arginine | **100** |
| Mannitol | **80** |
| Fatty acids | **60** |
| Flavour | **15** |
| Silicon dioxide | **5** |
| Sucralose | **1.5** |
| Acesulfame k | **0.85** |
| Neohesperidine DC | **0.16** |
| Total per tablet | **1600** |

| **EXAMPLE N5** | |
|---|---|
| PHARMACEUTICAL FORM | **EFFERVESCENT OROSOLUBLE GRANULATE IN BIPARTITE SACHET** |
| | |

| INGREDIENTS | mg/sachet |
|---|---|
| **CHAMBER A** | |
| S-adenosyl-L-methionine disulfate p-toluensulfonate (SAMe) | **500** |
| of which SAMe ion | 250 |
| L-Arginine | **200** |
| Fatty acids | **100** |
| Silicon dioxide | **10** |

| **CHAMBER B** | |
|---|---|
| Mixture of lactic ferments (Lactobacillus helveticus Rosell-52, Bifidobacterium longum Rosell -175) | 6x10⁹ CFU |
| Magnesium oxide | 186.6 |
| of which magnesium | 112,5 |
| Vitamin B6 (Pyridoxine Hydrochloride) powder | 11.53 |
| of which Vitamin B6 | 9.5 |
| Xylitol | **177.37** |
| Sodium Bicarbonate | **500** |
| Flavour | **25** |
| Silicon dioxide | **15** |
| Sucralose | **5** |
| Acesulfame k | **1** |
| Neohesperidine DC | **0.5** |
| Total per bipartite sachet | **2500** |

### 5.PILOT CLINICAL STUDY

A pilot clinical study entitled: "*Efficacy study of the food supplement based on S-adenosyl methionine (SAMe), and probiotics for the maintenance of the normal mood tone through the reduction of subthreshold depression, as risk factor of major depression"* was conducted. With this pilot study the efficacy of the composition of the invention based on S-adenosyl methionine (SAMe), *Lactobacillus helveticus e Bifidobacterium longum* as reported in Example 1 was evaluated in the improvement of the mood tone in healthy subjects, with a mild alteration of the mood tone, potential risk factor for the development of major depression. The duration of the treatment was two months.

The participants underwent two office visits. During the first visit, carried out at the enrollment (t0), sociodemographic, clinical parameters and scores obtained after the completion of the following two questionnaires, chosen as primary outcomes of the study, were recorded on the patient card (Case report form - CRF): 1) SCORE PATIENT HEALTH QUESTIONNAIRE-9 (PHQ-9), which investigates the presence "in the last two weeks" of the 9 symptoms of depression and the functional impairment caused by depression on the normal development of patient's life, and 2) HAMILTON RATING SCALE FOR DEPRESSION (HAM-D), which evaluates the severity of depressive symptoms and it is useful to document its modifications, e.g. following treatment. In the second visit, carried out after 10 weeks (t1), the above-mentioned questionnaires were again administered to the subjects and the obtained values were reported on the CRF.

In total 10 subjects were enrolled in the pilot clinical study, with the following inclusion and exclusion criteria:
INCLUSION CRITERIA: male and female subjects aged between 18 and 65 years with scores less than or equal to 19 in the HAM-D and to 17 in the PHQ-9 (corresponding to subjects with a slightly altered mood tone, unsuitable for antidepressant drugs because with mild or moderate depression), capable of understanding and satisfying the protocol requirements.

EXCLUSION CRITERIA: subjects severely depressed or who suffered from severe depression over the last years; individuals who took drugs or food supplements with activity on the psychological function over the 12 weeks before intake; pregnant women and those who took monoamine oxidase inhibitors (anti-MAO) over the 14 days before intake.

### Results

In Table 1 the demographic characteristics and scores at baseline of HAM-D and PHQ-9 questionnaires have been reported.

**Table 1**

| **Characteristics** | **Study population (n=10)** |
|---|---|
| Age | 39±19 |
| Ethnic origin: | European |
| HAM-D* | 14±5 |
| PHQ-9** | 10±4 |

| | |
|---|---|
| *The total score of the HAM-D to 21 questions was given by the sum of the first 17 items, considered those "cardinal" of depression: 0-7= subjects with normal mood; 8-16 = mild depression; 17-23= moderate depression; >24= severe depression **The score of the PHQ-9 was considered a range between 0 and 27. The scores between 5 and 9 indicates the presence of a subthreshold depression. The score of 10 is the optimal cut-off to highlight depressions of clinical relevance with three different levels of severity depending on the score. 0-4 = Absent; 5-9 = Subthreshold depression; 10-14 = Mild major depression; 15-19 = Moderate major depression; > 20 = Severe major depression. | |

In Table 2 the scores of HAM-D and PHQ-9 questionnaires recorded for each patient have been reported.

**Table 2**

| **Subject's code** | **Time** | **HAM-D** | **PHQ-9** |
|---|---|---|---|
| MRF | t0 | 18 | 11 |
| | t1 | 11 | 4 |
| MR | t0 | 16 | 12 |
| | t1 | 10 | 5 |
| AS | t0 | 7 | 9 |
| | t1 | 5 | 8 |
| AR | t0 | 19 | 17 |
| | t1 | 12 | 6 |
| A.S. | t0 | 13 | 6 |
| | t1 | 9 | 3 |
| ML | t0 | 9 | 6 |
| | t1 | 5 | 4 |
| MD | t0 | 14 | 6 |
| | t1 | 8 | 4 |
| TLAV | t0 | 19 | 7 |
| | t1 | 11 | 4 |
| ORRS | t0 | 12 | 10 |
| | t1 | 8 | 8 |
| GGED | t0 | 8 | 12 |
| | t1 | 2 | 8 |

**In** Table 3 the descriptive statistics (mean ± SD, range) of the score of the two questionnaires measured at t0 and t1 is reported.

**Table 3**

| | | |
|---|---|---|
| Questionnaires | t0 | t1 |
| HAM-D | 13±4 (7-19) | 8±3 (2-12) |
| PHQ-9 | 10±4 (6-17) | 5±2 (3-8) |

### Discussion

The results demonstrate that in all the enrolled subjects a reduction of the symptomatology of depressive state occurred.

As far as the HAM-D questionnaire is concerned, for the 60% of the subjects (6 out of 10) a decrease of the depressive state level was recorded. Specifically:
- 3 subjects out of 10 moved from a moderate depression state to a mild depression state;
- 3 subjects out of 10 moved from a mild depression state to the absence of depression;
- 4 subjects out of 10 remained in the same range of mild depression, even with a reduction of 6/4 points within the range;

As far as the PHQ-9 questionnaire is concerned, for the 90% of the subjects (9 out of 10) there was a decrease of the depressive state level. Specifically:
- 4 subjects out of 10 moved from a subthreshold depression state to the absence of depression;
- 4 subjects out of 10 moved from a mild depression state to a subthreshold depression state;
- 1 subject out of 10 moved from having a mild depression to the absence of depression;
- 1 subject out of 10 remained in the same range of "subthreshold depression", even with a reduction of 1 point withing the range.

With regard to tolerability and compliance, limited to the subjects enrolled in the pilot study, there were no side effects, the food supplement was well tolerated and no subject introduced changes of dosage.

### Conclusions

The results of the pilot study demonstrate how the composition of the invention based on S-adenosyl methionine (SAMe), *Lactobacillus helveticus* and *Bifidobacterium longum* is effective in reducing the depressive state or in restoring the normal mood tone, with optimal tolerability by the subjects who took the product.

## Claims

1. Orosoluble solid oral composition comprising the combination of S-adenosyl methionine with a mixture of probiotics consisting of Lactobacillus helveticus Rosell^{®}-52 and Bifidobacterium longum Rosell^{®}-175, in which said probiotics are present in the combination in a microencapsulated form, at least one physiologically acceptable excipient and an additional agent selected from a mixture of magnesium oxide and vitamin B6.

2. Orosoluble solid oral composition according to claim 1, for use in the treatment and/or prevention of mood disorders, preferably for the treatment of anxiety, depression, psychological stress, mild postpartum syndromes and/or stress related intestinal disorders.

3. Orosoluble solid oral composition for use according claim 2, wherein said S-adenosyl methionine is S-adenosyl methionine disulfate p-toluensulfonate.

4. Orosoluble solid oral composition according to claim 1 or orosoluble solid oral composition for use according to claim 2, wherein
said S-adenosyl methionine is contained in an amount from 50 to 500 mg, preferably from 100 to 400 mg, more preferably in an amount of about 200 mg (expressed in mg of SAMe ion).

5. Orosoluble solid oral composition for use according to claims 2-4, wherein said composition is in form of a tablet, capsule, powder, granule or granulate, preferably tablet or granulate, more preferably orosoluble granulate.

6. Orosoluble solid oral composition for use according to claims 2-5, wherein said depression is minor or subthreshold depression *(mild-depression).*

## Patentansprüche

1. Oroslösliche feste orale Zusammensetzung, umfassend die Kombination von S-Adenosylmethionin mit einer Mischung von Probiotika, bestehend aus Lactobacillus helveticus Rosell^{®}-52 und Bifidobacterium longum Rosell^{®}-175, wobei die Probiotika in der Kombination in mikroverkapselter Form vorliegen, mindestens einen physiologisch verträglichen Hilfsstoff und ein zusätzliches Mittel, ausgewählt aus einer Mischung von Magnesiumoxid und Vitamin B6.

2. Oroslösliche feste orale Zusammensetzung nach Anspruch 1 zur Verwendung bei der Behandlung und/oder Vorbeugung von Stimmungsstörungen, vorzugsweise zur Behandlung von Angst, Depression, psychologischem Stress, leichten postpartalen Syndromen und/oder stressbedingten Darmerkrankungen.

3. Oroslösliche feste orale Zusammensetzung zur Verwendung nach Anspruch 2, wobei das S-Adenosylmethionin S-Adenosylmethionindisulfat-p-Toluolsulfonat ist.

4. Oroslösliche feste orale Zusammensetzung nach Anspruch 1 oder oroslösliche feste orale Zusammensetzung zur Verwendung nach Anspruch 2, wobei das S-Adenosylmethionin in einer Menge von 50 bis 500 mg, vorzugsweise von 100 bis 400 mg, bevorzugter in einer Menge von etwa 200 mg (ausgedrückt in mg SAMe-Ion) enthalten ist.

5. Oroslösliche feste orale Zusammensetzung zur Verwendung nach den Ansprüchen 2-4, wobei die Zusammensetzung in Form einer Tablette, einer Kapsel, eines Pulvers, Körnchen oder eines Granulats, vorzugsweise einer Tablette oder eines Granulats, bevorzugter eines oroslöslichen Granulats, vorliegt.

6. Oroslösliche feste orale Zusammensetzung zur Verwendung nach den Ansprüchen 2-5, wobei die Depression eine geringfügige oder unterschwellige Depression (leichte Depression) ist.

## Revendications

1. Composition orale solide orosoluble comprenant la combinaison de S-adénosyl méthionine avec un mélange de probiotiques composé de Lactobacillus helveticus Rosell^{®}-52 et Bifidobacterium longum Rosell^{®}-175, dans laquelle lesdits probiotiques sont présents dans la combinaison sous une forme microencapsulée, au moins un excipient physiologiquement acceptable et un agent supplémentaire choisi parmi un mélange d'oxyde de magnésium et de vitamine B6.

2. Composition orale solide orosoluble selon la revendication 1, à utiliser dans le traitement et/ou la prévention des troubles de l'humeur, de préférence pour le traitement de l'anxiété, de la dépression, du stress psychologique, des syndromes post-partum légers et/ou des troubles intestinaux liés au stress.

3. Composition orale solide orosoluble selon la revendication 2, dans laquelle la S-adénosyl méthionine est la S-adénosyl méthionine disulfate p-toluensulfonate.

4. Composition orale solide orosoluble selon la revendication 1 ou composition orale solide orosoluble à utiliser selon la revendication 2, dans laquelle ladite S-adénosyl méthionine est contenue dans une quantité allant de 50 à 500 mg, de préférence de 100 à 400 mg, plus préférentiellement dans une quantité d'environ 200 mg (exprimée en mg d'ion SAMe).

5. Composition orale solide orosoluble à utiliser selon les revendications 2 à 4, dans laquelle ladite composition se présente sous la forme d'un comprimé, d'une capsule, d'une poudre, d'une granule ou d'un granulé, de préférence d'un comprimé ou d'un granulé, plus préférentiellement d'un granulé orosoluble.

6. Composition orale solide orosoluble à utiliser selon les revendications 2 à 5, dans laquelle ladite dépression est mineure ou inférieure au seuil (dépression légère).
